# EUROPEAN PATENT APPLICATION

(11) **EP 2 147 908 A1**
(43) Date of publication of application: **27.01.2010**
(21) Application number: 08733968.5
(22) Date of filing: 14.04.2008
(51) Int. Cl.: C07C 7/148, B01J 20/02, B01J 20/04, B01J 20/10

(54) **A PROCESS FOR PURIFYING HBr IN HYDROCARBONS**

(30) Priority: 13.04.2007 CN 200710034727
(71) Applicant: Microvast Technologies, Ltd., Zhejiang 313000 (CN)
(72) Inventor: ZHOU, Xiaoping, Zhejiang 313000 (CN); LIU, Zhen, Hunan, 410082 (CN); Li, Wensheng, Hunan, 410082 (CN); REN, Yanqun, Hunan, 410082 (CN)
(74) Representative: Tomlinson, Edward James
(86) International application number: PCT/CN2008/000770
(87) International publication number: WO 2008/125016

(57) **Abstract**

The present invention provides a method for separating HBr from HBr-containing hydrocarbons (alkenes, aromatics and/or alkanes) to purify the hydrocarbons. Reacting a silica supported metal oxide solid material (MOₓ/SiO₂, MOₓ=MgO, CoO, Co₂O₃, CuO and mixture thereof) with HBr in HBr-containing hydrocarbons, so as to ensure that the concentration of HBr is reduced to below 1.87×10⁻¹⁶, and then oxidizing the solid material which reacted with HBr in oxygen or air to regenerate MOₓ/SiO₂, meanwhile Br₂ is recycled. Therefore, the purpose of continuous purification of hydrocarbons can be achieved.

## Description

### FIELD OF THE INVENTION

The present invention relates to a method of separating HBr from hydrocarbons, especially from alkenes. This invention belongs to the field of chemical separation method of HBr from hydrocarbons.

### BACKGROUND OF THE INVENTION

The separation of HBr from hydrocarbons is seldom needed in the related field, so there are few related reports or patents. In our recent research, we designed a new process for conversion of methane or other lower hydrocarbons into higher hydrocarbon products, especially alkenes. For example, CN 200610031377.9 discloses a process as the following:

In the first step, namely reaction (A), low hydrocarbons, such as methane, react with HBr/H₂O and oxygen over catalyst A to produce alkyl alkyl bromides, of which the reaction formula is shown below.

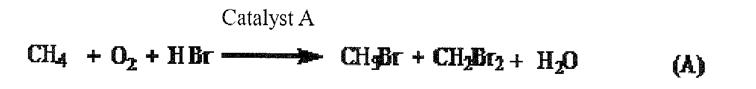

In the second step, namely reaction (B), alkyl bromides transform into high hydrocarbonssuch as alkenes, aromatics, and small alkanes, and HBr. The conversion of alkyl bromides can be higher than 99%. The reaction formula is shown below. 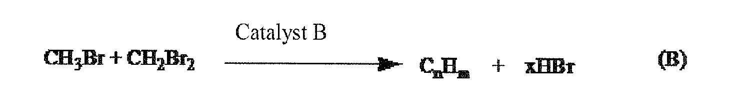 **n, m, x =2,3,4,5,6,7,9,9,10,11,12,13**

HBr will be produced in the reaction B. The HBr can be roughly washed using conventional methods, e.g. by feeding the HBr-containing hydrocarbons into the dilute HBr solution from reaction A to absorb the HBr. However, the certain amount of HBr remains in the products. Because of Lewis base property of alkenes, it is difficult to separate HBr from alkenes and aromatics completely by the conventional method.

### DISCLOSURE OF THE INVENTION

The present invention discloses a purification method for HBr-containing hydrocarbons which comprise alkenes, aromatics and alkanes.

In the purification method, contacting the HBr-containing hydrocarbons with a silica supported metal oxide solid that is formulated as MOₓ/SiO₂, where the metal oxide reacts with HBr to produce metal bromide. Then the metal bromide is oxidized by oxygen or oxygen of air to regenerate metal oxide on the support and the resultant Br₂ is recycled at the same time.

In this process, HBr-containing hydrocarbons pass through a containerfilled with MOₓ/SiO₂ and perform contacting by folwing through at a certain temperature (100∼600 °C). The metal oxide MOₓ reacts selectively with HBr to give non-volatile metal bromide and water to complete the purification of hydrocarbons. When the MOₓ/SiO₂ reaches saturation absorbing HBr, the feedstock of HBr-containing hydrocarbons was switched to another container filled with MOₓ/SiO₂ carrying out the same process.

The containerfilled with MOₓ/SiO₂ absorbing HBr was purged by steam and then oxygen or air was fed into it to regenerate metal oxide at a certain temperature (250∼600 □) and recycle bromine simultaneously. Two or more coordinate containers filled with MOₓ/SiO₂ operate purification-regeneration cycle to reach the purpose of purifying HBr-containing hydrocarbons and recovering bromine.

According to the present invention, MOₓ/SiO₂ filled in the purification container is a silica supported compound selected from the group consisting of MgO, CoO, Co₂O₃, CuO and mixture thereof. The silica is commercial silica or prepared from silicon-containing precursor, such as silicates, SiCl₄ or silica ester. The silica support was soaked in the solution comprising one or more soluble salts of acetate, nitrate and/or bromide of Mg, Co or Cu to give a mixture, then the mixture was dried and calcined to obtain the solid material of MOₓ/SiO₂.

In the operation of absorbing HBr, when contacting the HBr of hydrocarbons and MOₓ/SiO₂ the reaction between HBr and metal oxide MOₓ (MgO, CoO, Co₂O₃, CuO and mixture thereof) of MOₓ/SiO₂ is carried out at a temperature of from about 100° to about 600° preferably from about 150° to about 400°, more preferably from about 170° to about 300°. After absorbing HBr, the step of regenerating MOₓ/SiO₂ is carried out at a temperature of from about 250° to about 600°, preferably from about 300° to about 500°, more preferably between about 320° and about 450°.

For performing the invention method to purifying the HBr-containing hydrocarbons, the system comprises 2-8 MOₓ/SiO₂ filled containers connected by switch valve. Every container filled with MOₓ/SiO₂ can be a columnar fixed bed purification tower, which two ends are connected with switch valves. In a complete process, four steps of purifying, purging, regenerating and purging are carried out by switch valve. When the fixed bed purification tower is in the stage of absorbing, the inlet switch valve of purification tower is switched to HBr-containing hydrocarbons feedstock and the outlet switch valve of purification tower is switched to hydrocarbons tank. When the absorption of HBr reaches saturation, the hydrocarbons in the bed must be purged by steam and then oxygen could be fed into for regeneration. So the next step is purging stage. When the fixed bed purification tower is in the stage of purging, the inlet switch valve of purification tower is switched to the steam and the outlet switch valve of purification tower is switched to a gas-liquid separation tank, and the outlet of separation tank is connected to HBr-containing hydrocarbons. When the residual hydrocarbons is purged and the regeneration can be performed. In the stage of regenerating, the inlet switch valve of purification tower is switched to oxygen or air supplying system and the outlet switch valve of purification tower is switched to a Br₂ storage tank. After regeneration, next operation can not be performed because there is some oxygen in the system. A purging process should be need to blow away the oxygen in the purification tower before next purification operation. In this operation the inlet switc valve of purification tower is switched to the steam and the outlet switch is switched to vent. On the operation process, the performing is not consecutive for every container, so at least two or more same purification tower are used to ensure consecutive purification of HBr-containing hydrocarbons. When a tower is in the stage of purifying, other towers are in the other three operating stages, so the complete process can be consecutively operated.

### BEST MODE FOR CARRYING OUT THE INVENTION

### Preparation of metal oxide(s)/silica (MOₓ/SiO₂)

### Silica:

Silica can be commercial silica, or prepared from SiCl₄ or silica ester. 120.0 mL of SiCl₄ was added into 800 mL of water and stirred at ambient temperature for 12 h to get water-containing silica gel. Drying the water-containing silica gel at 120° for 6 h and calcining at 450° for 4 h to get silica. The silica was crushed and sieved to particles between 40 and 60 mesh.

### MOₓ/SiO₂:

Adding 20.00 g of the silica particles and 99.25 mmol of dissoluble acetate, nitrate and/or bromide of M into 100 mL deionized water under stirring, impregnating at ambient temperature for 2 h and then drying at 120° for 6 h, finally calcining at 450° for 4 h to get the MOₓ/SiO₂ which was then crushed and sieved to particles between 40 and 60 mesh.

The MOₓ in the MOₓ/SiO₂ solid material was selected from the group consisting of MgO, CoO, Co₂O₃, CuO and their mixtures.

Purification of HBr-containing hydrocarbons:
A: propylene
B: methane (75%), isobutene (10.0%), propylene (1.0%), cyclopentene (6.0%), cyclohexane (3.0%), benzene (2.0%), toluene (1.0%) and xylene (2.0%).

Heating a quartz tube filled with quartz sand up to 200°, pumping 40wt% of HBr/H₂O solution into the quartz tube at a rate of 3.0 mL/h, meanwhile A was directed into the quartz tube at a rate of 5.0 mL/min, the outlet of which was connected to a gas-liquid separation tank, to give a final outlet gas that is HBr-containing mixed gas. The outlet gas was introduced into AgNO₃ solution (4.0 M, 5.0 mL) for determining the concentration of HBr in mixed gas. The concentration of HBr in mixed gas was 5.3mol%.

The resultant mixed gas was directed into a reaction tube filled with 10.0g of MgO/SiO₂ solid material at 200°, and the outlet gas from the reaction tube was introduced into AgNO₃ solution(4.0 M, 5.0 mL) for determining the concentration of residual HBr. AgBr was produced when the MOₓ was exhausted. The volume of mixed gas was recorded at this time. The reaction tube was switched from HBr/C₃H₆ to steam and purged for 5 min. The solid material in the reaction tube was then regenerated by oxygen (or air) at 320∼450° until no bromine was flowed out, when the reaction tube was switched back to steam and purged for 5min to complete a cycle process.

After three cycles, the system reached stable. Every gram of MgO/SiO₂ can be applied for purification of 0.375 L of propylene that was equal to absorption of 0.1 g of HBr. The concentration of Br⁻ is 1.4×10⁻¹³ M when AgBr is appeared 7.0×10⁻¹⁶ mol of Br⁻ was contained in the 5.0 mL of AgNO₃ solution (4.0M). These Br⁻ was arised from 3.75 L propylene, so the residual concentration of HBr in mixed gas was less than 1.87×10⁻¹⁶ mol/L.

The same result was obtained when purifying the mixture of B and HBr.

## Claims

1. A method for separating HBr from HBr-containing hydrocarbons, comprising:
a) providing a container and a solid material composed of at least one metal oxide and a support therein;
contacting HBr-containing hydrocarbons with the solid material at a certain temperature; reacting the metal oxide with HBr to produce metal bromide and water; directing the hydrocarbons into a storage tank;
b) switching HBr-containing hydrocarbons to another similar container when metal oxide exhausts, to perform the process a);
purging first container by steam;
directing oxygen or air into first container, regenerating metal oxide at a certain temperature and recycling Br₂;
c) connecting two or more such containers by switch valve and thereby forming a purification system, cycling the process a) and process b) and HBr is separated from HBr-containing hydrocarbons.

2. A method as claimed in claim 1, wherein said hydrocarbons are alkanes, alkenes and/or aromatics.

3. A method as claimed in claim 1, wherein said metal oxide is selected from MgO, CoO, Co₂O₃, CuO and mixture thereof.

4. A method as claimed in claim 3, wherein said metal oxide is prepared by soaking the support in the solution of one or more acetate, nitrate and/or bromide of Mg, Co and Cu, and then drying and calcining them.

5. A method as claimed in claim 1, wherein said support is silica.

6. A method as claimed in claim 1, wherein said silica is commercial silica or prepared from silicates, SiCl₄ or silica ester.

7. A method as claimed in claim 1, wherein the step of contacting HBr-containing hydrocarbons with the solid material is carried out at a temperature of between about 100° and about 600°.

8. A method as claimed in claim 7, wherein the step of contacting HBr-containing hydrocarbons with the solid material is carried out at a temperature of between about 150° and about 400°.

9. A method as claimed in claim 8, wherein the step of contacting HBr-containing hydrocarbons with the solid material is carried out at a temperature of between about 170° and about 300°

10. A method as claimed in claim 1, wherein the step of regenerating metal oxide is carried out at a temperature of between about 250° and about 600°.

11. A method as claimed in claim 10, wherein the step of regenerating metal oxide is carried out at a temperature of between about 300° and about 500°.

12. A method as claimed in claim 11, wherein the step of regenerating metal oxide is carried out at a temperature of between about 320° and about 450°.

13. A method as claimed in claim 1, wherein the purification system is composed of 2-8 containers filled with the solid material connected by switch valve.

14. A method as claimed in claim 13, wherein the containers are columnar fixed bed reactors.
